(19) **Europäisches Patentamt**
**European Patent Office**
**Office européen des brevets**

(11) **EP 2 208 068 B1**

(12) **EUROPÄISCHE PATENTSCHRIFT**

(45) Veröffentlichungstag und Bekanntmachung des Hinweises auf die Patenterteilung:
**17.04.2013 Patentblatt 2013/16**

(21) Anmeldenummer: **08848943.0**

(22) Anmeldetag: **13.11.2008**

(51) Int Cl.:
*G01N 33/564* (2006.01)

(86) Internationale Anmeldenummer:
**PCT/DE2008/001894**

(87) Internationale Veröffentlichungsnummer:
**WO 2009/062497 (22.05.2009 Gazette 2009/21)**

(54) **VERFAHREN ZUR ENDTITERBESTIMMUNG UND DESSEN AUSWERTUNG MITTELS INDIREKTEN IMMUNFLUORESZENZ TEST**

METHOD FOR END-TITRE DETERMINATION AND THE EVALUATION THEREOF BY MEANS OF AN INDIRECT IMMUNOFLUORESCENCE ASSAY

PROCÉDÉ DE DÉTERMINATION DU TITRE FINAL ET D'ÉVALUATION DE CELUI-CI AU MOYEN D'UN TEST D'IMMUNOFLUORESCENCE INDIRECT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **13.11.2007 DE 102007054792**

(43) Veröffentlichungstag der Anmeldung:
**21.07.2010 Patentblatt 2010/29**

(73) Patentinhaber: **Medipan GmbH**
**15827 Dahlewitz/Berlin (DE)**

(72) Erfinder: **HIEMANN, Rico**
**01561 Thiendorf, Ortsteil Sacka (DE)**

(74) Vertreter: **Boeckh, Tobias et al**
**HERTIN**
**Anwaltssozietät**
**Kurfürstendamm 54/55**
**10707 Berlin (DE)**

(56) Entgegenhaltungen:
**WO-A1-2005/101291     DE-A1- 19 801 400**

- **HOLLINGSWORTH PETER N ET AL: "Precise quantitation of antinuclear antibodies on HEp-2 cells without the need for serial dilution" CLINICAL AND DIAGNOSTIC LABORATORY IMMUNOLOGY, Bd. 3, Nr. 4, 1996, Seiten 374-377, XP002517115 ISSN: 1071-412X**
- **HERR J C ET AL: "AN ELISA FOR MEASURING ANTISPERM AUTOANTIBODIES FOLLOWING VASECTOMY IN LEWIS RATS" AJRIM (AMERICAN JOURNAL OF REPRODUCTIVE IMMUNOLOGY AND MICROBIOLOGY), Bd. 11, Nr. 3, 1986, Seiten 75-81, XP008102830 ISSN: 8755-8920**

**Beschreibung**

Die Erfindung betrifft ein Verfahren zur Endtiterbestimmung bei der Bestimmung von Antikörpern gegen nukleäre und zytoplasmatische Antigene in Humanen Seren mittels indirekten Immunfluoreszenz Test. Offenbart wird darüber hinaus einen Kit zur in-vitrö Diagnostik zur Bestimmung von Antikörpern gegen nukleäre und zytoplasmatische Antigene in Humanen Seren mittels indirekten Immunfluoreszenz Test. Die Erfindung betrifft auch ein Computerprogramm zur Auswertung und zur Bestimmung des Endtiters im Rahmen des genannten Verfahrens.

HINTERGRUND DER ERFINDUNG

Autoimmunkrankheiten sind Krankheiten, deren Ursache eine überschießende Reaktion des Immunsystems gegen körpereigenes Gewebe ist. Irrtümlicherweise erkennt das Immunsystem körpereigenes Gewebe als zu bekämpfenden Fremdkörper. Dadurch kommt es zu schweren Entzündungsreaktionen, die zu Schäden an den betroffenen Organen führen. Für die Erkennung von Fremdstoffen sind T-Zellen verantwortlich. Im Thymus werden sie dafür geschult, nur an MHC-Moleküle anzudocken und körpereigene Strukturen zu tolerieren. Bei Autoimmunkrankheiten verhalten sich diese Zellen entgegen ihrer Natur. Anstatt eindringende Fremdkörper abzuwehren, greifen sie körpereigene Strukturen an. Organe und Gewebe werden als fremd empfunden, die lebensnotwendig für den Organismus und das Immunsystem sind. Das Immunsystem richtet seine ganze Abwehrstärke gegen diese Strukturen (zelluläre, wie auch humorale Abwehrreaktionen, in deren Folge Autoantikörper gebildet werden), was zur Folge hat, dass diese Organe und Gewebe im Laufe der Zeit ihre Funktion aufgeben müssen. Die Erfindung zielt auf die Diagnostik und damit spätere Behandlung von Autoimmunerkrankungen ab.

Grundsätzlich ist eine serologische Charakterisierung von Autoimmunerkrankungen durch den Nachweis von Autoantikörper-Profilen möglich. Die Mehrheit dieser Antikörper richtet sich gegen nukleäre und zytoplasmatische Antigene. Mit den Erkrankungen des rheumatischen Formenkreises sind vorwiegend Antinukleäre Antikörper (ANA) assoziiert. Einige dieser ANA sind krankheitsspezifisch und werden als diagnostische Marker benutzt. Dazu gehören beispielsweise Antikörper gegen:

- Doppelstrang-DNA (ds-DNA) und das Sm-Antigen beim systemischen Lupus erythematodes (SLE)

- Fibrillarin bei der Sklerodermie, Topoisomerase I (Scl-70) bei der diffusen Sklerodermie, Centromere (ACA) beim CREST

- Histidyl-tRNA-Synthetase (Jo-1) bei der Polymyosits

- PM-Scl bei der Überlappung zwischen Polymyosits und Sklerodermie.

ANA mit unterschiedlicher Prävalenz finden sich bei mehreren Erkrankungen. Dazu gehören: Anti-Histon-Antikörper beim SLE, medikamentös induzierten Lupus und bei der nutritiv toxischen chronischen Lebererkrankung: Anti-RNP-Antikörper beim SLE und Sharp Syndrom (MCTD: mixed connective tissue disease) und Anti-SS-A (Ro) und Anti-SS-B (La) Antikörper beim SLE und Sjörgen Syndrom. Antimitochondriale Antikörper (AMA) des Anti-M2 Typs reagieren mit Proteinen des Alpha-Ketosäure-Dehydrogenase Komplexes der Mitochondrien und sind charakteristische Marker für die primär billiäre Zirrhose (PBC), einer chronisch cholestatischen Lebererkrankung.

Die früheste Methode zum Nachweis von ANA und AMA ist der Immunfluoreszenztest (IFT), wobei Gewebegefrierschnitte oder Einzelzellen als Substrat verwendet werden. Dabei ist die unterschiedliche Speziesspezifität der nachzuweisenden Antikörper ein wesentliches Kriterium. Für einige humane Antikörper konnte gezeigt werden, dass sie ausschließlich mit Gewebe vom Menschen oder Primaten reagieren, während andere Autoantikörper Spezies-unspezifisch auch auf Gewebeschnitten von Ratte, Maus, Kaninchen oder Meerschweinchen reagieren. Die jeweiligen Antigene unterscheiden sich also im Hinblick auf ihre phylogenetische Entwicklung. Die mehr Spezies-unspezifischen Antigene bleiben im Lauf der Evolution stärker konserviert und finden sich daher auch bei weiter entfernten Spezies. Der Nachteil, dass nicht alle tierischen Zellen zum Nachweis bestimmter Autoantikörper geeignet sind, kann bei der Verwendung von HEp-2 Zellen vernachlässigt werden. Bei den sogenannten HEp-2 Zellen handelt es sich um eine humane Larynx-Epithelioma Zellinie, die eine hohe Spezifität für die meisten humanen Autoantikörper gegen nukleäre Antigene (ANA/ENA) aufweist (Hollingworth et al., Clin. Diagn. Lab. Immunol. Vol.3, 1996 374-377). Systemische rheumatisch-entzündliche Erkrankungen wie z.B. der Systemische Lupus erythematodes (SLE) und seine Varianten, die Progressive Systemische Sklerose (PSS), das Primäre Sjögren Syndrom, die Dermatomyositis, das Sharp Syndrom (mixed connective tissue disease - MCTD) oder die Rheumatoide Arthritis (RA) sind durch das Auftreten einer Reihe von Autoantikörpern charakterisiert, die gegen Komponenten des Zellkerns und des Zytoplasmas gerichtet sind. Obwohl die ätiopathogenetische Bedeutung dieser Autoantikörper nicht vollständig geklärt ist, können sie als Marker verschiedener Krankheitsbilder sowie als Aktivitätsparameter eingesetzt werden (Tan E. M., Adv Immunol 1982, 33:167-240; Tan E.M., Adv Immunol. 1989, 44: 93-151).

Eine geeignete Methode in der Autoantikörperdiagnostik ist heutzutage der so genannte indirekte Immunfluoreszenz Test. Der Immunfluoreszenztest auf HEp-2 Zellen ist ein sensitiver Screeningtest zur Bestimmung von Antinukleären Antikörpern (ANA), der über die Erkennung von Fluoreszenzmustern eine Aussage auf bestimmte zugrundeliegende Antigene und assoziierte Erkrankungen zulässt (Moore et al., Cancer Res. 1955, 15: 998-602; Weller et al., Proc. Soc. Exp. Biol. Med. 1954, 86: 789-794). Beim indirekten Immunfluoreszenz Test (IIF/IFT) werden HEp-2 Zellen einer humanen Epithelzelllinie als Substrat verwendet, die eine hohe Sensitivität für die meisten humanen Autoantikörper gegen nukleäre Antigene (ANA/ENA) haben. HEp-2 Zellen (humane Epithelzellen) werden von unterschiedlichen Herstellern derzeit bereitgestellt (z.B. INOVA Diagnostics, San Diego, USA; Kallestadt, Chaska, USA; Immuno Concepts, Sacramento, USA).

Ein indirekter ANA HEp-2 Immunfluoreszenztest zur qualitativen und semi-quantitativen ANA Bestimmung läuft wie folgt ab: Die Antikörper in verdünnten Patientenproben und Kontrollen reagieren im ersten Reaktionsschritt spezifisch mit den Antigenen der auf einem Objektträger fixierten HEp-2 Zellen. Nicht-gebundene Komponenten werden nach 30 Minuten Inkubation bei Raumtemperatur durch einen Waschschritt entfernt. Die gebundenen Antikörper reagieren im zweiten Reaktionsschritt spezifisch mit anti-human-Antikörpern (IgG und leichtkettenspezifisch), die an Fluorescein-Isothiocyanat (FITC) gekoppelt sind. Überschüssige Konjugatmoleküle werden nach 30 Minuten Inkubationszeit bei Raumtemperatur von den an der festen Phase gebundenen Immunkomplexen durch einen weiteren Waschschritt getrennt. Nach dem Eindecken werden die Objektträger unter einem Fluoreszenzmikroskop (Anregungswellenlänge 490 nm, Emissionswellenlänge 520 nm) manuell abgelesen. Entsprechend der histologischen Anordnung der Antigene in der HEp-2 Zelle ergeben sich spezifische Fluoreszenzmuster.

Eines der größten Probleme beim indirekten Immunfluoreszenz Test ist die Auswertung fluoreszenzoptischer Bilder in der Autoantikörperdiagnostik an HEp-2-Zellen. Nach heutigem Stand der Technik weist ein automatisiertes Verfahren in der Autoimmundiagnostik mittels HEp-2-Zellen im wesentlichen folgende Merkmale auf: Ein System zur Bilderfassung mittels Fluoreszenzmikroskop und Digitalkamera, bestehend aus einer automatischen Bildanalyse und Ermittlung von Fluoreszenzmuster beschreibenden Merkmalen, einer automatischen Klassifikation der Fluoreszenzmuster und der Ausgabe des erkannten Musters an das Labordatensystem. Als Aufnahmeeinheit dient beispielsweise ein Fluoreszenzmikroskop mit Kamera und einem Standard-PC. Das aus der Messung resultierende Fluoreszenzmuster ermöglicht derzeit die Erkennung von 7 verschiedenen Grundmustern (homogen, nukleolär, fein gesprenkelt, grob gesprenkelt, Zentromer, peripher; multiple nukleäre Punkte).

Ein solches System ist beispielsweise aus der DE 198 01 400 C1 bekannt. Die DE 198 01 400 C1 beschreibt ein Verfahren und eine Anordnung zur automatischen Erkennung, Eigenschaftsbeschreibung und Interpretation von HEp-2-Zellmustern. Dieses Verfahren und die entsprechende Anordnung dienen dem Nachweis von Autoimmunerkrankungen, wobei über eine zweidimensionale Aufnahme und Digitalisierung, Aufteilung in den Hintergrund und das Bild der geschnittenen HEp-2-Zellen, Einteilung in eine Anzahl diskreter Klassenbilder, Zusammenfassung der Bildpunkte zu einzelnen Objekten, Bestimmung von Merkmalen für die Objekte, Vergleich der Zellmuster und Anzeige und/oder Speicherung des Zellmusters und der zugeordneten Klassenzugehörigkeit die Interpretation der HEp-2-Zellmuster erfolgt. Die Anordnung nach der DE 198 01 400 C1 besteht aus einer Aufnahmevorrichtung und einer bildsegmentierenden, einer in Klassenbilder einteilenden, einer Merkmale bestimmenden und einer die Zellmuster vergleichenden Anordnung. Die Anordnungen sind in einem datenverarbeitenden Computer nacheinander enthalten und verknüpft. Ein ähnliches System wird auch in der EP 1 733 333 B1 beschrieben.

Die vorgenannten Methoden beruhen auf dem Prinzip der Endpunkt-Titration, die eine semiquantitative Methode zur Bestimmung einer Antikörpermenge in einem Serum ist. Bei dieser Methode wird eine fortlaufende Verdünnung des Serums und somit des Antikörpers mit einem konstanten Volumen getestet. Das Ergebnis wird als reziproker Wert der höchsten Verdünnungsstufe angegeben, bei der noch ein Immunfluoreszenzmuster zu sehen war. Das Problem dabei ist, dass ein qualitativ positives Testergebnis allein noch keinerlei fundierte diagnostische Aussage ermöglicht und zulässt. Erst die semiquantitative Bestimmung, dass bedeutet ein Austitrieren der Sera mit Angabe des Endpunktiters, führt zu einer diagnostisch relevanten Aussage.

Allerdings muss hierbei eingeschränkt werden, dass eine klinische Diagnose nicht unproblematisch ist. Denn bis zu 10% der normalen Bevölkerung können ANA aufweisen. Das Auftreten von ANA ist abhängig von Alter und Geschlecht der Patienten, dabei nimmt die Häufigkeit mit steigendem Alter zu. Ein positives Ergebnis mit niedrigem Titer ohne Vorliegen klinischer Symptome kann deshalb bei älteren Menschen als normal angesehen werden. Gesunde junge Menschen sind dagegen in der Regel ANA negativ. SLE Patienten unter corticosteroider Therapie können ebenfalls ANA negativ sein. ANA können auch bei Verwandten von Patienten mit Bindegewebserkrankungen auftreten, die später ebenfalls erkranken können.

Gegenwärtig erfolgt die Auswertung von Fluoreszenzmustern aufgrund der unterschiedlichen Fluoreszenzintensität einzelner Auftragstellen, die entsprechend einer Empfehlung des Center for Desease Control and Prevention (CDC), Atlanta, USA, wie folgt unterteilt werden (Lyerla and Forrester: The Immunofluorescence (IF) test. In: Immunofluorescence methods in virology, USDHHS, Georgia, 1979, 71-81):

| | |
|---|---|
| 4+ | = maximale Fluoreszenz, brillant gelb-grün |
| 3+ | = weniger brillante gelb-grüne Fluoreszenz |
| 2+ | = eindeutige, aber matte gelb-grüne Fluoreszenz |
| 1+ | = sehr schwache unterdrückte Fluoreszenz |

Die Intensität der Fluoreszenz spiegelt jedoch nicht die Antikörperkonzentration wider. Unterschiede zwischen Optik, Filtern und Lichtquellen bei verschiedenen Mikroskopen können zu Unterschieden in der Fluoreszenzintensität von mehr als einer Stufe führen. Insofern kommt es zu so genannten "negativen" und "positiven" Ergebnissen. Eine Probenverdünnung wird als "ANA negativ" bewertet, wenn die HEp-2 Zellen eine Fluoreszenz kleiner 1+ und das Fehlen eines bestimmbaren Musters aufweisen. Eine Probenverdünnung wird hingegen als "ANA positiv" bewertet, wenn die HEp-2 Zellen eine Fluoreszenz von 1+ oder größer sowie ein klar bestimmbares Fluoreszenzmuster aufweisen.

Die Bestimmung des Titrationsendpunktes ist mithin auch abhängig von Typ und Beschaffenheit des Fluoreszenzmikroskopes, von der Vergrößerung des Objektives sowie der subjektiven Beurteilung des Betrachters. Mit Bakterien verunreinigte Proben sowie Waschpufferlösungen können zu unspezifischen Anfärbungen des Zellsubstrates führen.

Bei einer semi-quantitativen Titration wird die Verdünnungsstufe als Ergebnis angegeben, bei der letztmalig eine 1+ Fluoreszenz abgelesen wird. Diese Titerstufe wird als Endpunktiter für das Serum angegeben. Der Titer ist der reziproke Wert der Verdünnungsstufe. Bei der vom Center for Desease Control and Prevention (CDC) empfohlenen vierfachen Verdünnungsreihe kann der Endpunkt der Titration wie folgt extrapoliert werden:

| | |
|---|---|
| 1:40 | = 3+ |
| 1:160 | = 2+ |
| 1:640 | = +/- |
| 1:2560 | = - |

Der extrapolierte Titer ist demnach 1: 320.

Dabei gelten Titer von 40 und 80 als niedrig positiv-klinisch nicht relevant, 160 und 320 werden als mittlere Titer angesehen und könnten klinisch relevant sein, Titer von 640 und größer gelten als hoch positiv und sind klinisch relevant. Derartige Verdünnungsreihen herzustellen und entsprechende Versuche durchzuführen ist jedoch aufwendig und auch sehr kostenintensiv; In der Praxis wird daher aus Kostengründen häufig auf die vierfache Verdünnungsreihe verzichtet und man beschränkt sich auf eine oder zwei Verdünnungen (Bsp: 1:80 und 1:320).

Im Stand der Technik wird beispielsweise eine folgenden Anordnung zur Durchführung einer Titerbestimmung verwendet: Die verwendete Anordnung weist das zu untersuchende Präparat auf, sowie die Verwendung eines motorisierten X-Y-Probentischs, ein motorisiertes Fluoreszenzmikroskop (mit Z-Steuerung) einschließlich einer steuerbaren Kamera und letztlich einen Personalcomputer mit entsprechender Software und dem Zugriff auf eine Datenbank.

| | |
|---|---|
| Tag 1: | Eingangsscreening mit 1:80 (+ 1:320); Verdünnung mit visueller Abschätzung des Endtiters erfolgt durch einen Medizinisch technischen Assistenten (MTA); |
| Tag 2: | Verdünnung mit 1:640, 1:1280 und gegebenenfalls weiteren Stufen bis 1 über und unter dem geschätzten Endtiter; |

Wenn der Endtiter nicht gefunden werden konnte, dann sind weitere Verdünnungen notwendig, bis das Fluoreszenzmuster nicht mehr sichtbar ist.

Das bei dieser Methode auffallende Problem ist, dass die Fluoreszenzintensität des Präparates von vielen Faktoren abhängig ist, wie beispielsweise der Empfindlichkeit der konkret eingesetzten Kamera, dem Färbungsprotokoll, dem Antibleaching Medium, dem Anregungslicht, welches wiederum vom Alter des Brenners (der Lichtquelle) abhängig ist und den verwendeten optischen Komponenten wie Objektiv und Filtersatz.

Die Nachteile bei den gegenwärtig im Stand der Technik verwendeten Verfahren lassen sich daher wie folgt zusammenfassen:

• Die Auswertung von Fluoreszenzmustern erfolgt nach wie vor rein subjektiv durch den Betrachter.

• Die Intensität von Fluoreszenzmustern unterliegt großen Schwankungen, da diese abhängig sind von vielen apparativen Parametern wie beispielsweise der Empfindlichkeit der konkret eingesetzten Kamera, dem Färbungsprotokoll, dem Antibleaching Medium, dem Anregungslicht, welches wiederum vom Alter der Lichtquelle abhängig ist und den verwendeten optischen Komponenten der Analyseoptik. Letztlich wird aber die Intensität und "Helligkeit" eines Fluoreszenzmusters von jedem Betrachter anders wahrgenommen. Der subjektive Einfluss der "Helligkeitsabschätzung" ist mithin nicht zu vernachlässigen.

- Die Auswertung selbst wird auf das im Labor machbare und als zumeist notwendig erachtete Minimum beschränkt, d.h. es werden nur unvollständige Mess- oder Verdünnungsreihen durchgeführt und auf einen Endtiterpunkt extrapoliert, der nur nach der Erfahrung des jeweiligen Benutzers mehr oder weniger genau ist und somit unter oder auch über dem Endtiter liegen kann. "Richtige" Ergebnisse bleiben oft dem Zufall überlassen.

- Die subjektive auf phänotypischen Merkmalen beruhende Auswertung der Fluoreszenzmuster führt wiederum zu einer erhöhten Fehlerquote und damit sogar zu einer Fehlinterpretation der auszuwertenden Bilder. Dies führt letztlich in der diagnostischen Praxis dazu, dass entweder Autoimmunerkrankungen nicht als solche erkannt werden, oder das falsch-positive Resultate ermittelt und ausgewertet werden. In der diagnostischen und klinischen Praxis führt dies zu möglicherweise unterlassenen Behandlungen oder aber zu verfrühten Behandlungen; beides stellt für den Patienten eine mehr als unbefriedigende Situation dar.

## DETAILLIERTE BESCHREIBUNG DER ERFINDUNG

Aus diesem Stand der Technik resultiert daher das zu lösende technische Problem, welches in einer objektiven und reproduzierbaren Auswertung von Fluoreszenzmustern in der Autoantikörperdiagnostik mittels indirekten Immunfluoreszenz Test zu sehen ist.

Gelöst wird diese Aufgabe durch die Merkmale der selbstständigen Ansprüche, sowie der jeweiligen Unteransprüche.

Erfindungsgemäß ist danach ein Verfahren zur Endtiterbestimmung bei der Bestimmung von Antikörpern gegen nukleäre und zytoplasmatische Antigene in Humanen Seren mittels indirekten Immunfluoreszenz Test vorgesehen, umfassend mehrere Verfahrensschritte, wobei eine Reaktion und Bindung von in Patientenseren enthaltenen Autoantikörpern mit und an Antigene von auf einem Objektträger fixierten HEp-2 Zellen, Leukozyten, Crithidia luciliae und/oder Gewebeschnitten stattfindet. Es findet eine spezifische Fluoreszenzmarkierung der gebundenen Autoantigene statt, gefolgt von einer fluoreszenzmikroskopischen Untersuchung der auf dem Objektträger gebundenen fluoreszenzmarkierten Autoantikörper, sowie optische Aufnahme und Auswertung der fluoreszenzoptischen Bilder anhand der Fluoreszenzintensität in einem Auswertungssystem. Das letztgenannte Auswertungssystem wird zuvor, d.h. vor der Bindung von in Patientenseren enthaltenen Autoantikörpern mit und an Antigene von auf einem Objektträger fixierten HEp-2 Zellen, Leukozyten, Crithidia luciliae und/oder Gewebeschnitten mittels wenigstens zweier Kontrollseren mit definiertem Titer zur Erstellung einer Verdünnungsreihe kalibriert, wobei zudem auch die Intensität des Anregungslichtes gemessen wird. Die Fluoreszenzintensität der aufgenommenen fluoreszenzoptischen Bilder wird zu dem Titer der Kontrollseren in Relation gesetzt wird, woraus sich der Endtiter des zu untersuchenden Patientenserums ergibt. Der Endtiter des zu untersuchenden Patientenserums ergibt sich aus der ermittelten Referenzfunktion des einkalibrierten Systems, die abhängig vom Muster bzw. der Musterkombination linear oder nicht-linear sein kann.

Das erfindungsgemäße Verfahren der Endtiterbestimmung zeichnet sich demnach durch eine Kalibrierphase, sowie die Messung der Intensität des Anregungslichtes aus; ferner ist die maximal sinnvolle Belichtungszeit, d.h. die Endbelichtungszeit, sowie der Eingangstiter des Patientenserums und die Belichtungszeit der Kamera von Bedeutung.

Wesentliches Merkmal der Erfindung ist ein eigener Kalibriervorgang der verwendeten Optik zur Auswertung der Fluoreszenzmuster. Dies wird erreicht durch Messung des Anregungslichtes der Fluoreszenz und mit einem erfindungsgemäßen Objektträger, der auf seiner Oberfläche mehrere Kontroll-Seren mit definiertem Titer aufweist, über die das optische System kalibriert wird. Zur Kalibrierung wird eine Messung der mittleren Belichtungszeit über mehrere Bilder für jedes definierte Kontrollserum durchgeführt und anschließend wird die Bestimmung des Endpunktes der Kamera vorgenommen, die sich wiederum aus der maximal sinnvollen Belichtungszeit ergibt, welche der Belichtungszeit bei Endtiter der Kontrollseren entspricht. Der Endtiter des zu untersuchenden Patientenserums ergibt sich also aus der Endbelichtungszeit, dem Eingangstiter des Patientenserums und der Belichtungszeit der

Kamera ergibt, insbesondere nach der ermittelten Kalibrierfunktion die im einfachsten linearen Fall nach folgender Gleichung berechnet werden kann

$$Serumtiter = \frac{Eingangstiter}{Belichtungszeit} * Endbelichtungszeit$$

Ferner wird offenbart ein Kit zur in-vitro Diagnostik zur Bestimmung von Antikörpern gegen nukleäre und zytoplasmatische Antigene in Humanen Seren mittels indirekten Immunfluoreszenz Test, umfassend zumindest

a. einen Objektträger mit mehren mit HEp-2 Zellen beschichteten Auftragsstellen zum Auftragen von Kontroll- und Patentenseren,

b. Kontrollseren mit unterschiedlichem, vordefiniertem Titer zur Kalibrierung des optischen Systems einer immunfluoreszenzmikroskopischen Mess- und Auswertanordnung,

c. fluoreszenzmarkierte anti-humane Antikörper zur spezifischen Kopplung von an HEp-2 Zellen gebundenen Antikörpern.

Der im Kit zur Anwendung kommende anti-humane Antikörper ist anti-human-Immunglobulin und kann gegebenenfalls mit Fluorescein-Isothiocyanat gekoppelt sein.

Es wird hiermit somit ein Kit beschrieben, das für die Durchführung des erfindungsgemäßen Verfahrens zur Endtiterbestimmung bei der Bestimmung von Antikörpern gegen nukleäre und zytoplasmatische Antigene in Humanen Seren geeignet ist. Vorteilhafterweise umfasst der Kit außerdem Reagenzien, Waschlösungen und Lösungen, die zur zweckbestimmten Durchführung angepasst sind. Vorzugsweise bietet dieser Kit ebenfalls ein Protokoll für jeden notwendigen Schritt zur in-vitro Diagnostik, sowie ggf. Referenzwert-Tabellen und Kalibrierungsinformationen. Ferner umfasst der Kit Informationen zum Kombinieren der Inhalte desselben.

Die Erfindung stellt somit einen Immunfluoreszenztest auf HEp-2 Zellen als sensitiven Screeningtest zur Bestimmung von Antinukleären Antikörpern (ANA) zur Verfügung, der über die Erkennung von Fluoreszenzmustern eine Aussage auf bestimmte zugrundeliegende Antigene und assoziierte Erkrankungen zulässt. Der Kit umfasst ein Reagenziensatz zur qualitativen und semi-quantitativen Bestimmung von Antikörpern gegen Antigene im Zellkern und im Zytoplasma von HEp-2 Zellen in humanem Serum mittels automatisierter Auswertung.

Darüber hinaus ist ein Computerprogramm Gegenstand der Erfindung, welches gespeichert in einem Computer lesbaren Medium umfassend Computer lesbare Daten (Programcode), durch die der Computer angewiesen wird, ein Verfahren gemäß der Erfindung bei laufendem Computerbetrieb auszuführen, wodurch ein Verfahren zur Endtiterbestimmung bei der Bestimmung von Antikörpern gegen nukleäre und zytoplasmatische Antigene in Humanen Seren mittels indirekten Immunfluoreszenz Test durch Auswerten fluoreszenzoptischer Bilder in der Autoantikörperdiagnostik mittels indirektem Immunfluoreszenz Test elektronisch gesteuert und ausgewertet werden kann.

Offenbart ist ferner eine erfindungsgemäße Anordnung zur Endtiterbestimmung in der Autoantikörperdiagnostik bei der Bestimmung von Antikörpern gegen nukleäre und zytoplasmatische Antigene in Humanen Seren mittels indirekten Immunfluoreszenz Test, umfassend

a. ein System zur Bilderfassung mittels eines Fluoreszenzmikroskops mit Kamera

b. ein System zur automatischen Bildanalyse und Ermittlung der erfassten Fluoreszenzmuster und Floureszenzintensitäten der auf einem Objektträger zur Reaktion und Bindung von in Patientenseren enthaltenen Autoantikörpern mit und an Antigene von auf dem Objektträger fixierten HEp-2 Zellen, Leukozyten, Crithidia luciliae und Gewebeschnitten.

Diese Anordnung weist den Vorteil auf, dass sowohl ein System zur Bilderfassung als auch zur gleichzeitigen automatischen Bildanalyse zur Verfügung gestellt wird. Dies verringert insbesondere die aus dem Stand der Technik bekannten nachteile der unzureichenden Analyse der Daten.

Das erfindungsgemäße Verfahren sowie der darauf basierenden Kit sind für zellbasierte Tests geeignet, bei denen die Muster und evtl. Titer automatisch ausgelesen werden. Es eignet sich zur qualitativen und semi-quantitativen Bestimmung von Antikörpern gegen Antigene im Zellkern und im Zytoplasma von HEp-2 Zellen in humanem Serum mittels automatisierter Auswertung. Neben HEp-2-Zellen sind auch Leukozyten, Crithidia luciliae und Gewebeschnitte bevorzugt.

Das Fluoreszenzsignal ist Schwankungen unterworfen, weswegen sich als vorteilhaft der Zusatz von Antibleaching-Reagenzien erwiesen hat, beispielsweise 2,3,5,6 Tetramethyl-1,4-Phenylendiamin ($C_{10}H_{16}N_2$).

Ein wesentliches Element der Erfindung, insbesondere des zugrundeliegenden Verfahrens ist mithin, dass die zu untersuchende Einheit "Zelle + Konjugat" konstant bleibt. Das Konjugat (= Farbstoff + Antikörper) wird durch eine Substanz, die das Ausbleichen (Antibleaching) des Farbstoffes verhindert, stabil gehalten. Bevorzugt wird dabei 2,3,5,6 Tetramethyl-1,4-Phenylendiamin ($C_{10}H_{16}N_2$) verwendet. Die grundlegenden technischen Parameter (Fluoreszenzfilter, Kamera, Objektiv) sind ebenfalls konstant. Daher kann lediglich die Belichtungsintensität schwanken, die daher zur Titervorhersage gemessen wird (= Messung des Anregungslichtes). Damit ist die einzigste variable Komponente der Technik die Lichtquelle, weswegen auch das Anregungslicht der Fluoreszenz gemessen wird, da Fluoreszenzanregung und Fluoreszenzemission miteinander korrelieren. Die Antikörperkonzentration (Titer) ergibt sich demnach aus der Belichtungszeit des Serumbildes.

Erfindungsgemäße Vorteile liegen mithin in einer objektiven Bestimmung des Endtiters, sowie in einer schnellern und kostengünstigen Erfassung des Endtiters des Serums. Ein universeller Einsatz des erfindungsgemäßen Systems ist auf verschiedenen Messsystemen möglich. Ferner sind unterschiedliche Präparate verwendbar (universell für verschiedene Präparate (Zellen, Einzeller, Gewebeschnitte) einsetzbar). Darüber hinaus werden durch die Erfindung Ressourcen technischer und finanzieller Art eingespart.

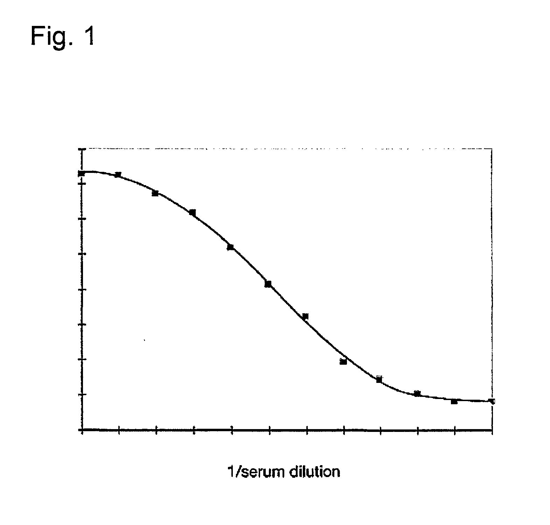

Weitere vorteilhafte Maßnahmen sind in den übrigen Unteransprüchen beschrieben; die Erfindung wird anhand von Ausführungsbeispielen und der nachfolgenden Figuren näher beschrieben, ohne aber auf diese Beispiele beschränkt zu sein; die Figuren 1 bis 4 zeigen unterschiedliche Kalibrierungskurven.

BEISPIELE

Die Erfindung wird nun näher auch in Hinblick auf die Figuren anhand von Ausführungsbeispielen beschrieben, ohne allerdings auf diese beschränkt zu sein.

I. Vorbereiten und Durchführung der Messungen. Hierzu wird folgendes Verfahren angewendet.

1. Mit mehreren Kontrollseren wird die Fluoreszenzoptik kalibriert.

2. Die Patientenseren werden bei Raumtemperatur auf die vorgesehenen Auftragstellen des beschichteten Objektträgers pipettiert und 30 Minuten bei RT in feuchter Kammer inkubiert.

3. Die Objektträger werden mit PBS Lösung abgespült und 2 x 5 min mit frischer PBS Lösung in einem Färbetrog gewaschen

4. Die Objektträger werden mit einer Konjugatlösung bedeckt und 30 Minuten bei Raumteperatur in einer feuchten Kammer, UV-lichtgeschützt inkubiert..

5. Wiederholung von Schritt 3..

6. Die Objektträger werden mit einem Deckglas blasenfrei abgedeckt und unter dem Fluoreszenzmikroskop vermessen.

II. Kalibrierung

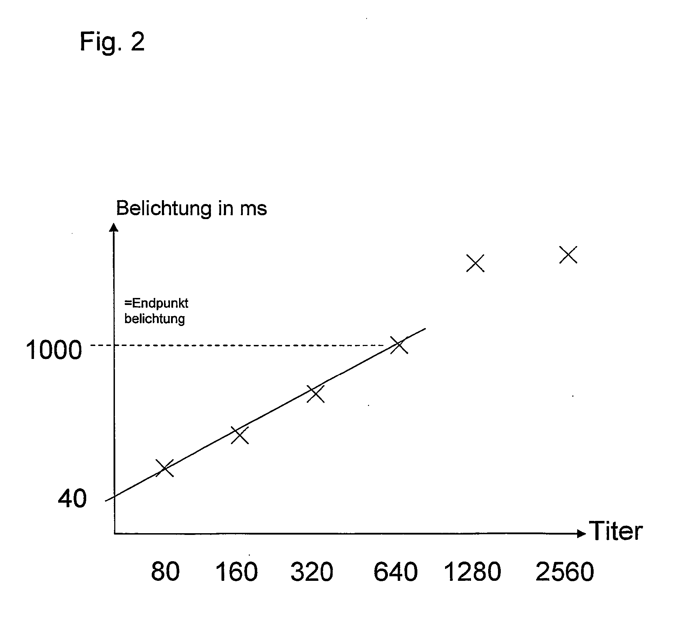

Figur 2 zeigt die Kalibrierung mit einem ersten Kalibrierserum 1, wobei der Endpunkt bekannt ist (640). Die Regression der Messpunkte der Belichtungszeiten für austitriertes Serum ist im einfachsten Fall eine Gerade (Y = m x + b), wobei m die Steigung und b der Schnittpunkt mit der Y-Achse ist. Komplexere Kurven lassen sich nach folgender Formel berechnen:

$$\mathrm{sig}(t) = \frac{1}{1 + c^{-t}}$$

Da der Endpunkt beim Kalibirierserum bekannt ist, sollte nur der Messpunkt bis zum Endpunkt in die Regression einbezogen werden, denn darüber hinaus wird nur die Autofluoreszenz des Gewebes gemessen.

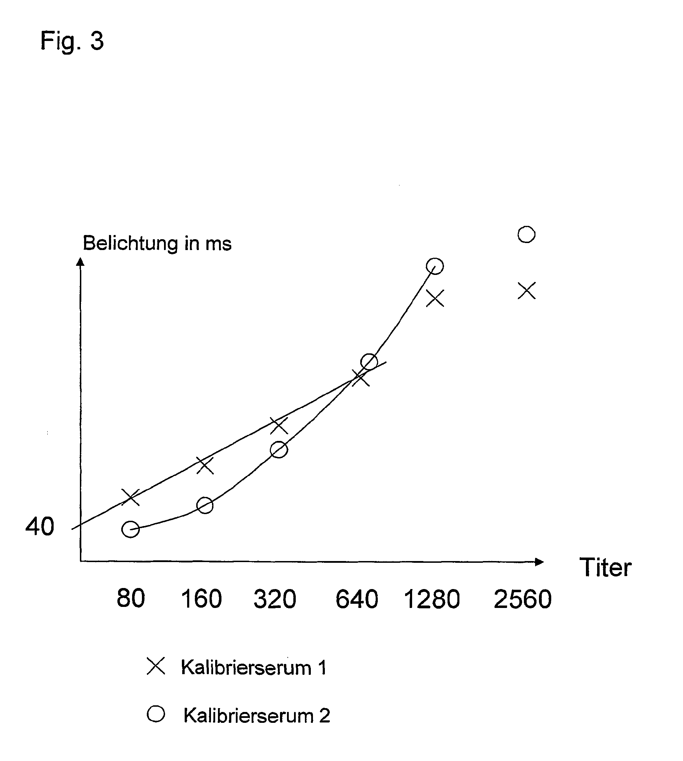

Figur 3 zeigt das (homogene) Kalibrierserum 1 mit bekanntem Endpunkt (640) in Korrelation zu einem zweiten Kalibrierserum 2 (Punktmuster) mit ebenfalls bekanntem Endpunkt (1280). Es können sich unterschiedliche Kurvenverläufe für die verschiedenen Kalibrierseren ergeben, abhängig vom Muster und der Spezifität (Linear-Gerade oder exponentiell oder sigmoidal). Jedes der Kalibrierseren hat eine andere Antikörperspezifität (homogenes Muster, Zentromer-Muster, Punktmuster, etc. (siehe unten)) In der Praxis wird bei der Messung das Muster ermittelt, die Kalibrierkurve herausgesucht und aus dem Einsetzen des Verdünnungstiters in die Kalibrierkurve der Endtiterpunkt des Serums berechnet.

III. Titerabschätzung aus dem Serum

Es wird eine HEp-2 End-Titerabschätzung mit einem Eingangstiter von 1:80 durchgeführt.

Der "Kalibrierungsobjektträger" wird wie folgt kalibriert (s. Beispiel I, Punkt 1.): Es wird ein erstes Serum mit einem bekannten Titer 1:640 in 8 Verdünnungen in 8 so genannten Wells auf dem Objektträger aufgetragen und mittels Fluoreszenzmikroskopie belichtet und vermessen. Gemessen wird bei unterschiedlichen Belichtungszeiten die Fluoreszenzintensität. Hierbei ergibt sich folgende Messreihe:

Tabelle 1 Kalibrierung des Objektträgers

| Well | Verdünnung | gemessene Belichtungszeit | Anmerkung |
|---|---|---|---|
| 1 | 1:40 | 290 ms | |
| 2 | 1:80 | 602 ms | |
| 3 | 1:160 | 1200 ms | |
| 4 | 1:320 | 2510 ms | |
| 5 | 1:640 | 5000 ms | Endpunkt |
| 6 | 1:1280 | 6100 ms | Sättigung (Autofluoreszenz des Präparates) |
| 7 | 1:2560 | 6105 ms | |
| 8 | 1:5120 | 6205ms | |

Aus den Werten der Wells 1 bis 5 wird durch Regression der wahre Funktionsverlauf bestimmt (vgl. hierzu Figur 1; Y-Achse: Lichtintensität; X-Achse: Verdünnung). In diesem Beispiel liegt die maximal sinnvolle Belichtungszeit demnach bei 5000 ms, da danach die Autofluoreszenz des Präparates einsetzt und somit die Ergebnisse verfälschen würde. Aufgrund der beschriebenen Kalibrierung des Objektträgers (Kalibrierungsobjektträger) ist in diesem Beispiel der Endpunkt des kalibrierten Systems mit 5000 ms bekannt.

Der Objektträger, der die Patientenprobe trägt (Patientenobjektträger), weist hingegen unterschiedlich Verdünnungen auf. Es ist beispielsweise die Verdünnung des Serums im Well mit 1:80 bekannt. Die Bilder werden automatisch so belichtet, dass das Signal der Immunfluoreszenz vollständig vom Sensor der Kamera erfasst wird (vgl. Hiemann et al. Cytometry Part A 69A (2005)). Gemessen wird eine mittlere Belichtungszeit der Kamera von 500 ms; dies ergibt sich aus der Mittelung der Belichtungszeiten einzelner Bilder des Wells. Die in der Praxis zu lösende Frage lautet demnach: Wie hoch ist der zu erwartende Endtiter des Serums?

Die auf der Erfindung basierende Lösung führt zu dem Ergebnis, dass - bei linearem Zusammenhang - sich der gesuchte Endtiter nach der ermittelten Referenzfunktion des einkalibrierten Systems berechnet. Gemäß dem obigem Beispiel also

$$Serumtiter = \frac{Eingangstiter}{Belichtungszeit} * Endbelichtungszeit = \frac{80}{500ms} * 5000ms = 800$$

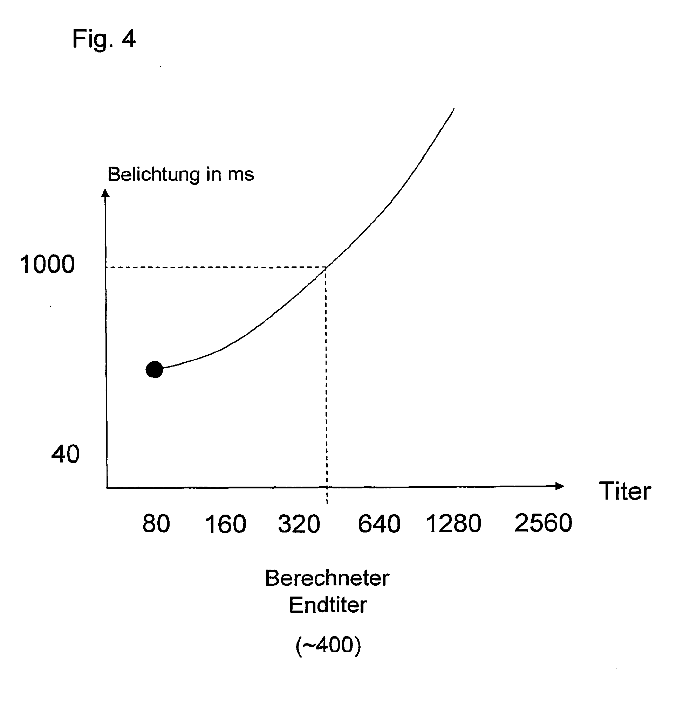

Figur 4 zeigt eine weitere Messung eines Patientenserums mit Punktmuster, wobei der Endpunkt unbekannt ist. Es wurde ein bekannter Verdünnungstiter von 80 verwendet. Die gemessene Belichtungszeit betrug 200ms. Daraus berechnet sich ein Endtiter durch Einsetzen in die Kalibrierfunktion von ~400.

Die nachfolgende Tabelle 2 gibt die Messergebisse zweier Testreihen (lineare Kalibrierfunktion) gemäß der Erfindung wieder:

Tabelle 2

| Test-reihe | Serum | Titer Software | Titer Mensch | Spezifität | Abweichung | Anmerkung |
|---|---|---|---|---|---|---|
| 1. | 2518 | 320 | 640 | dsDNA/SS-A | -1 | |
| | 2520 | 320 | 320 | dsDNA/SS-A | 0 | |
| | 2500 | 640 | 640 | SS-A/SS-B | 0 | |
| | 2507 | 2560 | 5120 | SS-A/SS-B | -1 | |
| | 2519 | 1280 | 1280 | RNP | 0 | |
| | 2522 | 320 | 320 | RNP | 0 | |
| | 2529 | 320 | 640 | Sm | -1 | |

(fortgesetzt)

| Test-reihe | Serum | Titer Software | Titer Mensch | Spezifität | Abweichung | Anmerkung |
|---|---|---|---|---|---|---|
| | 2514 | 1280 | 2560 | RNP | -1 | |
| 2. | 3698 | 1280 | 1280 | Scl-70 | 0 | |
| | 3699 | 640 | AMA 320, CENP 10240 | | -4 | bei sehr hohen Titern nicht mehr linear |
| | 3633 | 2560 | 5120 | Sm/RNP | -1 | |
| | 2423 | 2560 | 10240 | PmScl | -2 | bei sehr hohen Titern nicht mehr linear |
| | 3462 | 1280 | 1280 | SS-A/SS-B | 0 | |
| | 3007 | 2560 | 5120 | dsDNA/SS-A | -1 | |

Dabei bezeichnet "Serum" ein mit einer internen Referenznummer versehenes Patientenserum.

"Titer Software" bezeichnet den von der erfindungsgemäßen Software ermittelten Titer aufgrund der oben beschriebenen Verhältnismäßigkeit aus bekanntem Eingangstiter, verwendeter Belichtungszeit und Endbelichtungszeit.

"Titer Mensch" gibt den ermittelten Titer aufgrund der subjektiven Wahrnehmung der Fluoreszenzintensität gem. dem bislang üblichen Verfahren wieder.

"Abweichung" kennzeichnet eine Abweichung der subjektiv ermittelten Titerstufen. Dabei wird eine Abweichung (s. oben) von +/- 1 Titerstufe als nicht signifikant angesehen und spielt demnach in der Praxis keine Rolle.

Daraus ergibt sich das überraschende Ergebnis, dass es mit dem erfindungsgemäßen Verfahren möglich ist, wesentlich exakter und fehlerfreier den Endtiter zu bestimmen.

Das erfindungsgemäße Verfahren, der darauf basierende beschriebene Kit, sowie das Testprinzip werden im Detail nachfolgend vorgestellt

a. Der erfindungsgemäße Kit umfasst zumindest folgende Bestandteile:

- Objektträger mit Auftragstellen, die mit HEp-2 Zellen beschichtete sind, ggf. schutzgasversiegelt,

- Waschpuffer/Probenverdünner PBS Puffer, PH 7,4 ± 0,1, als Festsubstanz,

- Konjugat enthaltend anti-human-Immunglobulin (IgG- und leichtkettenspezifisch), gekoppelt mit FITC,

- Eindeckmedium permanent Glyzerinlösung, phosphatgepuffert, mit Antifadingreagenz,

- Deckgläser

- Positive Kontrolle Positives Humanserum mit Antikörperspezifität,

- Negative Kontrolle Negatives Humanserum

- Messsystem zur Ablesung und Bewertung der Fluoreszenzmuster

Ferner sind übliche Hilfsmittel vorgesehen wie erstellbare Mikropipetten (10, 100, 1000 µl), Pipettenspitzen, Probenverdünnungsröhrchen, Messzylinder oder Maßkolben, eine feuchte Kammer, Plastikwaschflasche, Färbetröge.

b. Die Antikörper in verdünnten Patientenproben oder Kontrollseren reagieren in einem ersten Reaktionsschritt spezifisch mit den Antigenen der auf dem Objektträger fixierten HEp-2 Zellen. Nicht-gebundene Komponenten werden nach 30 Minuten Inkubation bei Raumtemperatur durch einen Waschschritt entfernt. Die gebundenen Antikörper reagieren in einem zweiten Reaktionsschritt spezifisch mit anti-human-Antikörpern (IgG und leichtkettenspezifisch), die an Fluorescein-Isothiocyanat (FITC) gekoppelt sind. Überschüssige Konjugatmoleküle werden nach 30 Minuten Inkubationszeit bei Raumtemperatur von den an den festen Phase gebundenen Immunkomplexen durch einen weiteren Waschschritt getrennt. Entsprechend der histologischen Anordnung der Antigene in der HEp-2 Zelle sind spezifische Fluoreszenzmuster erkennbar. Nach dem Eindecken werden die Objektträger unter einem Fluoreszenzmikroskop (Anregungswellenlänge 490 nm, Emissionswellenlänge 520 nm) mit einem automatisierten Meßsystem abgelesen.

c. Zur Probengewinnung lässt man das durch Venenpunktion entnommene Blut von Patienten gerinnen und isoliert anschließend das Serum durch Zentrifugation. Vor dem Einsatz im Test werden die Seren auf Raumtemperatur gebracht und ggf. wird durch kurzes Aufschütteln eine entsprechende Homogenität gesichert. Die Patientenproben für den erfindungsgemäßen Test werden im Verhältnis 1:80 (v/v) mit PBS Puffer verdünnt. Zusätzlich zu dieser Screeningverdünnung kann eine 1:320 Verdünnung zur Absicherung der Titervorhersagen sowie zur besseren Bewertung bei eventuell auftretenden Mischmustern eingesetzt werden (EASI Empfehlung). Ausgehend von der 1:80 (v/v) Verdünnung werden die Proben 4-fach mit PBS Pufferlösung weiter verdünnt, z. B. 100 $\mu$l Probenverdünnung + 300 $\mu$l PBS Puffer.

d. Der erfindungsgemäße Test wird wie folgt durchgeführt:

d.1. Die Testreagenzien werden auf Raumtemperatur (RT, 20-25 °C) gebracht, die Objektträger werden erst unmittelbar vor Gebrauch aus Verpackung entnehmen und gekennzeichnet um Kontaminierungen zu vermeiden.

d.2. Pipettieren von 25 $\mu$l Kontrollen (25 $\mu$l der verdünnten Patientenseren)

d.3. Objektträger 30 Minuten bei Raumtemperatur in feuchter Kammer inkubieren.

d.4. Objektträger mit PBS Lösung abspülen.

d.5. 2 x 5 min mit frischer PBS Lösung in je einem Färbetrog waschen.

d.6. Objektträger einzeln entnehmen, PBS gut abtropfen lassen, 1 Tropfen Konjugat auf jede Auftragstelle geben, Auftragstelle dabei komplett bedecken.

d.7. Objektträger 30 Minuten bei Raumtemperatur in feuchter Kammer inkubieren. Dabei vor direktem Licht schützen.

d.8. Wiederholung von c.4 und c.5.

d.9. Objektträger einzeln entnehmen, PBS abtropfen lassen, je einen kleinen Tropfen Eindeckmedium auf den Rand jeder Auftragstelle geben. Deckglas vorsichtig so auf den Objektträger setzen, dass das Eindeckmedium eine blasenfreie geschlossene Schicht bildet.

d.10. Objektträger mittels des automatisierten Systems ablesen. Boden des Objektträgers von unten gut abwischen!

e. Bewertung der Ergebnisse

Das erfindungsgemäße automatisierte Auswertesystem liefert pro Auftragstelle eine Entscheidung positiv-negativ sowie ein Ergebnis hinsichtlich der Hauptfluoreszenzmuster und einen Vorschlag für den Endtiter (Konzentration des Antikörpers). Vom System positiv bewertete Proben können am PC anhand gespeicherter Bilder kontrolliert werden.
Eine Probe wird als ANA negativ bewertet, wenn die Intensität der Fluoreszenz in der 1:80 Verdünnung kleiner als ein von der Software vorgegebener Schwellenwert ist. Eine Probe wird als ANA positiv bewertet, wenn die Intensität der Fluoreszenz in der 1:80 Verdünnung größer als ein von der Software vorgegebener Schwellenwert ist.
Bei positiven ANA Ergebnissen erfolgt durch die Software, die Bestandteil der Erfindung ist, eine Eingruppierung des Fluoreszenzmusters in folgenden Gruppen: homogen, granulär, nukleolär, centromer, nuclear dots, Mitose, Zytoplasma.
Entsprechend der Anfärbung des Zellkernes der HEp-2 Zellen können verschiedene Muster unterschieden werden:

e.1. Homogen: Diffuse Anfärbung des gesamten Zellkernes, mit oder ohne Verschleierung der Nukleoli. Speziell in der Nähe des Endpunktes kann das Muster bei einigen Proben granulär wirken. Die Chromosomenregion von Mitosezellen zeigt eine stark positive Fluoreszenz. Antigene: DNA, Histone. Klinische Relevanz: hochtitrig spezifisch für SLE, in niederen Titern auch bei Rheumatoider Arthritis; Histon-Antikörper sind sehr stark assoziiert mit medikamenteninduziertem Lupus.

e.2. Peripher: Glatte Anfärbung der äußeren Bereiche des Zellkernes, schwächere Fluoreszenz im Inneren; nicht alle Zellen einer Auftragstelle müssen diese periphere Färbung zeigen, einige Zellen können ein homogenes Muster aufweisen. Die Chromosomenregion von Mitosezellen zeigt eine stark positive Fluoreszenz. (eine schmale ringförmige Anfärbung mit negativer Chromosomenregion in Mitosezellen rührt dagegen von Antikörpern gegen die Kernmembran). Antigene: DNA, Histone. Klinische Relevanz: hochtitrig in der aktiven Phase des SLE, niedrige Titer auch bei anderen Bindegewebserkrankungen.

e.3. gesprenkelt: Fluoreszierende Sprenkel über den gesamten Zellkern, sehr feine bis sehr grobe Sprenkel in Abhängigkeit des Antikörpertyps möglich. Die Chromosomenregion von Mitosezellen reagiert üblicherweise negativ.

a) Sm und nRNP :grobe Sprenkel, Aussparung der Nukleoli, Chromosomenregion der Mitosezellen negativ. Klinische Relevanz: Sm Antikörper sind hochspezifische Marker für SLE; hohe anti-nRNP Titer sind charakteristisch für MCTD, zusammen mit anderen ANAs bei SLE, RA, PSS.

b) SS-A und SS-B: kleine gleichförmige Sprenkel in gleichmäßiger Verteilung, Chromosomenregion der Mitosezellen negativ. Klinische Relevanz: sehr häufig beim primären Sjögren Syndrom, weniger häufig bei SLE, anti-SS-A sehr häufig bei neonatalem Lupus und kongenitalem Herzblock.

c) Scl-70: feine dichte Sprenkel mit Fluoreszenz der Nukleoli, Chromo-somenregion der Mitosezellen positiv. Klinische Relevanz: anti-Scl-70 gelten als Marker für PSS.

d) PCNA: variierend feine und grobe Sprenkel in 30-60% der Zellen, Mitosezellen positiv oder negativ. Klinische Relevanz: anti-PCNA treten in einer kleinen Prozentzahl von SLE Patienten auf.

e.4. Centromer: Diskrete Sprenkel über den gesamten Zellkern, die Anzahl entspricht dem einfachen oder multiplen Chromosomensatz. Das Fluoreszenzmuster der Mitosezellen folgt der Verteilung der Chromosomen: paarweise Punkte in der Äquatorialebene während der Metaphase, Auseinanderbewegung zu den Centrosomen während der Anaphase. Ein ähnliches Muster (multiple nuclear dots) wird durch NSP-1 (SP100) Antikörper verursacht, Chromosomenregion der Mitosezellen bleiben hier jedoch negativ. Antigene: Centromerproteine der Chromosomen. Klinische Relevanz: Marker für das CREST Syndrom, seltener bei diffuser Sclerodermie und Raynaud Phänomen.

e.5. Nukleolär: Fluoreszenz der Nukleoli innerhalb des Zellkernes, scharf abgegrenzt vom nicht angefärbten Kernplasma. Die Fluoreszenz der Nukleoli kann homogen oder gesprenkelt ("clumpy") sein. Oft von einem gesprenkelten Muster begleitet. Antigene: PMScl, RNA Polymerase I, Fibrillarin. Klinische Relevanz: hochtitrig spezifisch für PSS, Polymyositis-Dermatomyositis Overlap, niedrigtitrig bei SLE, Sjögren Syndrom, Raynaud Phänomen.

e.6. Spindelapparat: Netzwerk von feinen Fäden, die die Zentrosomen in Mitosezellen miteinander verbinden. Antigene: Spindelapparat in Mitosezellen. Klinische Relevanz: seltenes Muster bei einer Reihe von autoimmunen und anderen Erkrankungen (RA, SLE, PBC, Carpal Tunnel Syndrom).

e.7. Zytoplasma: Granuläre oder fädige Fluoreszenz im Zytoplasma

a) ribosomale RNP: feingranuläre Fluoreszenz im gesamten Zytoplasma, oft begleitet von einem nukleolären Muster (Bestätigung auf anderen Gewebeschnitten wird empfohlen). Klinische Relevanz: charakteristisch für einige Fälle bei SLE.

b) Jo-1 (PL-7, PL-12): feine Sprenkel mit allgemein schwacher Fluoreszenz, hauptsächlich im perinukleären Bereich. Klinische Relevanz: Polymyositis, Dermatomyositis.

c) mitochondrial: kleine gleichförmige Sprenkel in faserartiger Anordnung, dichter in kernnahen Bereichen (Bestätigung auf anderen Gewebeschnitten wird empfohlen). Klinische Relevanz: Marker für primär-biliäre Zirrhose (PBC)

d) Zytoskelett: faserige, spinnennetzartige Fluoreszenz über das Zytoplasma, verursacht durch Antikörper gegen Aktin und andere Bestandteile des Zytoskeletts (Vimentin, Tubulin), Bestätigung auf anderen Gewebe¬schnitten wird empfohlen. Klinische Relevanz: verschiedene, anti-Aktin häufig bei autoimmuner Hepatitis und Infektionserkrankungen

Das automatisierte Auswertesystem liefert pro Auftragstelle eine Entscheidung positiv-negativ sowie ein Ergebnis hinsichtlich der Hauptfluoreszenzmuster und einen Vorschlag für den Endtiter (Konzentration des Antikörpers). Vom System positiv bewertete Proben können am PC anhand gespeicherter Bilder kontrolliert werden.

**Patentansprüche**

1.  Verfahren zur objektiven Endtiterbestimmung bei der Bestimmung von Antikörpern gegen nukleäre und zytoplasmatische Antigene in Humanen Seren mittels indirekten Immunfluoreszenz Test, umfassend folgende Verfahrensschritte

    a. Reaktion und Bindung von in Patientenseren enthaltenen Autoantikörpern mit und an Antigene von auf einem Objektträger fixierten HEp-2 Zellen, Leukozyten, Crithidia luciliae und/oder Gewebeschnitten,
    b. spezifische Fluoreszenzmarkierung der gebundenen Autoantigene,
    c. fluoreszenzmikroskopische Untersuchung der auf dem Objektträger gebundenen fluoreszenzmarkierten Autoantikörper, sowie
    d. optische Aufnahme und Auswertung der fluoreszenzoptischen Bilder anhand der Fluoreszenzintensität in einem Auswertungssystem,
    **dadurch gekennzeichnet, dass**
    e. es sich um ein automatisiertes Auswertungssystem handelt, wobei

    i. der Optik des Auswertungssystems vor Durchführung des Verfahrensschrittes a. mittels a) wenigstens zweier Kontrollseren mit definiertem Titer zur Erstellung einer Verdünnungsreihe und b) Messung des Anregungslichts der Fluoreszenz des Systems kalibriert wird, und
    ii. die Fluoreszenzintensität der aufgenommenen fluoreszenzoptischen Bilder zu dem Titer der Kontrollseren rechnerisch in Relation gesetzt wird, woraus der Endtiter des zu untersuchenden Patientenserums berechnet wird.

2.  Verfahren nach Anspruch 1, wobei die spezifische Fluoreszenzmarkierung durch fluoreszenzmarkierte anti-human-Antikörper erfolgt.

3.  Verfahren nach Anspruch 1 oder 2, wobei zur Stabilisierung des Fluoreszenzsignals Antibleaching-Reagenzien zugesetzt werden.

4.  Verfahren nach Anspruch 3, wobei es sich bei dem Antibleaching-Reagenz um 2,3,5,6 Tetramethyl-1,4-Phenylendiamin handelt.

5.  Verfahren nach wenigstens einem der Ansprüche 1 bis 4, wobei sich der Endtiter des zu untersuchenden Patientenserums aus der Endbelichtungszeit, dem Eingangstiter des Patientenserums und der Belichtungszeit der Kamera ergibt, insbesondere nach der ermittelten Kalibrierfunktion, die im einfachsten linearen Fall nach folgender Gleichung berechnet werden kann:

$$Serumtiter = \frac{Eingangstiter}{Belichtungszeit} * Endbelichtungszeit$$

6.  Ein ausführbares Computerprogramm, gespeichert in einem computerlesbarem Medium umfassend einen Programmcode als computerlesbare Daten, durch die der Computer angewiesen wird, den Endtiter aufgrund eines Verfahrens zur Endtiterbestimmung bei der Bestimmung von Antikörpern gegen nukleäre und zytoplasmatische Antigene in Humanen Seren mittels indirekten Immunfluoreszenz Test gemäß einem der Ansprüche 1 bis 5 bei laufendem Computerbetrieb zu bestimmen.

7.  Computerprogramm nach Anspruch 6, wobei dieses zur automatischen Bildanalyse und Ermittlung der erfassten Fluoreszenzmuster und Fluoreszenzintensitäten der auf einem Objektträger zur Reaktion und Bindung von in Patientenseren enthaltenen Autoantikörpern mit und an Antigene von auf dem Objektträger fixierten HEp-2 Zellen, Leukozyten, Crithidia luciliae und/oder Gewebeschnitten geeignet ist.

## Claims

1. Method for objective end-titre determination in the determination of antibodies against nuclear and cytoplasmic antigens in human sera by means of an indirect immunofluorescence assay, comprising the following steps

    a. reaction and binding of autoantibodies contained within patient sera with and to antigens of HEp-2 cells, leukocytes, Crithidia luciliae and/or tissue sections, which are fixed to a slide,
    b. specific fluorescent marking of the bound autoantigens,
    c. fluorescent microscopic analysis of the fluorescently marked autoantibodies bound to the slide, in addition to
    d. optical recording and evaluation of the fluorescent optical images using the fluorescence intensity in an evaluation system,
**characterized in that**
    e. the evaluation system is an automated evaluation system, whereby

        i. the optics of the evaluation system are calibrated before carrying out the method steps by means of a) at least two control sera with defined titre for the preparation of a dilution series, and b) measurement of the excitation light of the fluorescence of the system, and
        ii. the fluorescence intensity of the recorded fluorescent optical images is computationally compared to the titre of the control sera, from which the end titre of the patient serum to be investigated is calculated.

2. Method according to claim 1, whereby the specific fluorescent marking is carried out with fluorescently marked anti-human antibodies.

3. Method according to claims 1 or 2, whereby anti-bleaching reagents are added to stabilize the fluorescence signal.

4. Method according to claim 3, whereby the anti-bleaching reagent is 2,3,5,6 tetramethyl-1,4-phenylenediamine.

5. Method according to at least one of the claims 1 to 4, whereby the end titre of the patient serum to be investigated results from the final exposure time, the input titre of the patient serum and the exposure time of the camera, preferably according to the determined calibration function, which in the simplest linear case can be calculated according to the following equation:

$$serum\ titre = \frac{input\ titre}{exposure\ time} * final\ exposure\ time$$

6. Executable computer programme, which is saved in a computer-readable-medium comprising a programme code as computer-readable-data, through which the computer is instructed, during active computer operation, to determine the end titre on the basis of a method for end-titre determination in the determination of antibodies against nuclear and cytoplasmic antigens in human sera by means of an indirect immunofluorescence assay according to claims 1 to 5.

7. Computer programme according to claim 6, whereby the computer programme is suited for automatic image analysis and determination of the captured fluorescent patterns and fluorescent intensities of autoantibodies from patient serum, that react with and are bound to antigens of the HEp-2 cells, leukocytes, Crithidia luciliae and/or tissue sections, which are fixed to the slide.

## Revendications

1. Procédé pour la détermination objective du titre final lors de la détermination d'anticorps dirigés contre des antigènes nucléaires et cytoplasmiques dans des sérums humains au moyen d'un test d'immunofluorescence indirecte, comprenant les étapes opératoires suivantes :

a. la réaction et la liaison d'anticorps contenus dans des sérums de patients avec et à des antigènes de cellules HEp-2, de leucocytes, de Chritidia luciliae et/ou de coupes histologiques fixés sur un porte-objet ;

b. le marquage spécifique par fluorescence des auto-antigènes liés;

c. l'analyse microscopique par fluorescence des auto-anticorps marqués par fluorescence liés sur le porte-objet ; et

d. l'enregistrement optique et l'évaluation des images optiques de fluorescence sur base de l'intensité de la fluorescence dans un système d'évaluation,

**caractérisé en ce que**

e. il s'agit d'un système d'évaluation automatisé, dans lequel

   i. l'optique du système d'évaluation est étalonnée avant la mise en oeuvre de l'étape opératoire a. au moyen a) d'au moins deux sérums témoins comprenant un titre défini pour l'établissement d'une dilution en série, et b) de la mesure de la lumière d'excitation de la fluorescence du système ; et

   ii. l'intensité de la fluorescence des images optiques de fluorescence enregistrées est mise en relation par calcul avec le titre des sérums témoins, calcul à partir duquel on calcule le titre final du sérum du patient à analyser.

2. Procédé selon la revendication 1, dans lequel le marquage spécifique par fluorescence a lieu via des anticorps antihumains marqués par fluorescence.

3. Procédé selon la revendication 1 ou 2, dans lequel on ajoute des réactifs s'opposant à la décoloration à des fins de stabilisation du signal de fluorescence.

4. Procédé selon la revendication 3, dans lequel en ce qui concerne le réactif s'opposant à la décoloration, il s'agit de la 2,3,5,6-tétraméthyl-1,4-phénylènediamine.

5. Procédé selon au moins une des revendications 1 à 4, dans lequel on obtient le titre final du sérum du patient à analyser à partir du temps de pose final, du titre de départ du sérum du patient et du temps de pose de la caméra, en particulier conformément à la fonction d'étalonnage déterminée que l'on peut calculer dans le cas linéaire le plus simple, conformément à l'équation suivante :

$$titre\ du\ sérum = \frac{titre\ de\ départ}{temps\ de\ pose} * temps\ de\ pose\ final$$

6. Programme informatique exécutable, mémorisé dans un support lisible par ordinateur comprenant un code programme à titre de données lisibles par ordinateur, par lequel l'ordinateur est invité à déterminer le titre final sur base d'un procédé pour la détermination du titre final lors de la détermination d'anticorps dirigés contre des antigènes nucléaires et cytoplasmiques dans des sérums humains au moyen d'un test d'immunofluorescence indirecte conformément à l'une quelconque des revendications 1 à 5, lorsque l'ordinateur est actif.

7. Programme informatique selon la revendication 6, ledit programme étant approprié pour l'analyse d'images et la détermination automatique du motif de fluorescence et des intensités de fluorescence enregistrés des auto-anticorps contenus dans des sérums de patients sur un porte-objet pour la réaction et la liaison avec et à des antigènes de cellules HEp-2, de leucocytes, de Chritidia luciliae et/ou de coupes histologiques fixés sur le porte-objet.

Fig. 1

1/serum dilution

## Fig. 2

Belichtung in ms

=Endpunkt
belichtung

1000

40

Titer

80    160    320    640    1280    2560

Fig. 3

X Kalibrierserum 1

O Kalibrierserum 2

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19801400 C1
- EP 1733333 B1

**In der Beschreibung aufgeführte Nicht-Patentliteratur**

- **HOLLINGWORTH et al.** *Clin. Diagn. Lab. Immunol.,* 1996, vol. 3, 374-377
- **TAN E. M.** *Adv Immunol,* 1982, vol. 33, 167-240
- **TAN E.M.** *Adv Immunol.,* 1989, vol. 44, 93-151
- **MOORE et al.** *Cancer Res.,* 1955, vol. 15, 998-602
- **WELLER et al.** *Proc. Soc. Exp. Biol. Med.,* 1954, vol. 86, 789-794